(19) Europäisches Patentamt
European Patent Office
Office européen des brevets

(11) EP 4 797 270 A1

(12) EUROPEAN PATENT APPLICATION
published in accordance with Art. 153(4) EPC

(43) Date of publication:
**26.08.2026 Bulletin 2026/35**

(21) Application number: **24880214.2**

(22) Date of filing: **21.10.2024**

(51) International Patent Classification (IPC):
***G16C 60/00*** *(2019.01)* ***G16H 50/30*** *(2018.01)*
***G16C 20/30*** *(2019.01)* ***G16C 20/90*** *(2019.01)*
***G01N 33/32*** *(2006.01)*

(86) International application number:
**PCT/KR2024/015998**

(87) International publication number:
**WO 2025/084877 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 KR 20230140690**

(71) Applicants:
- **HD Korea Shipbuilding & Offshore Engineering Co., Ltd.**
  **Seongnam-si, Gyeonggi-do 13553 (KR)**
- **HD Hyundai Heavy Industries Co., Ltd.**
  **Dong-gu**
  **Ulsan 44032 (KR)**
- **HD Hyundai Samho Co., Ltd.**
  **Jeollanam-do 58462 (KR)**

(72) Inventors:
- **HWANG, Kyoung Sub**
  **Seongnam-si Gyeonggi-do 13553 (KR)**
- **PARK, Chan Hyung**
  **Seongnam-si Gyeonggi-do 13553 (KR)**
- **SEO, Hwa Won**
  **Seongnam-si Gyeonggi-do 13553 (KR)**
- **SONG, Eun Ha**
  **Seongnam-si Gyeonggi-do 13553 (KR)**
- **LEE, Kyung Koo**
  **Ulsan 44032 (KR)**

(74) Representative: **Maiwald GmbH**
**Elisenhof**
**Elisenstraße 3**
**80335 München (DE)**

# (54) METHOD AND SYSTEM FOR SELECTING PAINT COMPOSITION

(57) A method for selecting a paint composition is disclosed. The method for selecting a paint composition comprises the steps of: receiving the components and amount of candidate raw materials of the paint composition through a user input interface; checking hypersensitivity component information about the candidate raw materials; calculating a skin hypersensitivity index of paint on the basis of the components and amount of the candidate raw materials and the hypersensitivity component information; evaluating the harmfulness of the paint to the skin on the basis of the skin hypersensitivity index of the paint; and selecting the paint composition on the basis of the evaluated harmfulness of the paint to the skin.

[Fig. 2]

START
S110
RECEIVE COMPONENT AND AMOUNT OF CANDIDATE RAW MATERIAL
S120
CHECK HYPERSENSITIVITY COMPONENT INFORMATION OF CANDIDATE RAW MATERIAL
S130
CALCULATE SKIN HYPERSENSITIVITY INDEX
S140
IS HARMFULNESS OF PAINT TO SKIN EVALUATED (IS LESS THAN REFERENCE HYPERSENSITIVITY INDEX?)
NO
YES
S150
SELECT PAINT COMPOSITION
END

EP 4 797 270 A1

## Description

### Technical Field

The present disclosure relates to a method and system for selecting a paint composition, and more particularly, to a method and system for selecting a paint composition, in which a skin hypersensitivity index is calculated based on the hypersensitivity and corrosiveness components in raw materials constituting a base material and a curing agent of the paint, and the paint is evaluated according to the skin hypersensitivity index, thereby selecting the paint composition.

### Background Art

The increasing use of chemicals in industrial settings enriches human life but also exposes people to new hazards and risks. Chemicals may be a cause of accidents and diseases. The harmfulness of chemicals to skin refers to the harmfulness of chemicals to a user's skin, and may manifest as skin disorders such as irritant contact dermatitis, allergic contact dermatitis, skin cancer, skin infections, and skin damage.

The shipbuilding industry uses paints on ships. Epoxy resins, etc., contained in these paints may be harmful to the skin. Therefore, users need to assess the potential harmfulness of the paint to skin before using the paint to determine the safety of the paint.

Currently, the skin harmfulness is assessed using Material Safety Data Sheets (MSDSs). However, these MSDSs indicate skin hypersensitivity for chemicals used in paints as either "1" or "None," regardless of the quantity of skin-hazardous substances included in the chemicals. Therefore, because the skin hypersensitivity is not specifically classified in MSDSs for paints, it is difficult to accurately assess the paint's harmfulness.

In addition, unexpected skin disorders often occur when paints are applied in industrial settings by referring only to Material Safety Data Sheets (MSDSs), without a separate verification of their skin harmfulness. Even when the skin harmfulness is tested, only fragmentary test methods (animal or alternative animal tests) are used, or referring only to partial results such as verifying only skin irritation among the test items may lead to unexpected skin disorders when applied in actual industrial sites (ships/construction/automobiles/other industrial painting).

Therefore, in order to accurately evaluate the harmfulness of the paint to the skin, there is a need to develop a method and system that quantify skin harmfulness based on the hypersensitivity information, the corrosiveness information, and the amount of substances contained in the paint components, and assign a risk grade to the paint according to the calculated skin harmfulness, thereby enabling an objective and systematic evaluation of the paint's risk grade. In addition, objectively and systematically evaluating the harmfulness of the paint may support the development of paint formulations with low skin irritation.

The above information disclosed in this Background section is only for enhancement of understanding of the background of the invention and therefore it may contain information that does not form the prior art that is already known in this country to a person of ordinary skill in the art.

### Disclosure of Invention

### Technical Problem

An aspect of the present disclosure is to provide a method and system for selecting a paint composition that calculates a skin hypersensitivity index based on hypersensitivity and corrosiveness components of raw materials constituting a paint's base material and curing agent, and evaluates the harmfulness of the paint to skin based on the skin hypersensitivity index, thereby enabling the selection of a suitable paint composition.

### Solution to Problem

According to an aspect of the present disclosure, a method for selecting a paint composition includes receiving components and amounts of candidate raw materials of the paint composition through a user input interface, checking hypersensitivity component information about the candidate raw materials, calculating a skin hypersensitivity index of paint based on the components and amounts of the candidate raw materials and the hypersensitivity component information, evaluating harmfulness of the paint to the skin based on the skin hypersensitivity index of the paint; and selecting the paint composition based on the evaluated harmfulness of the paint to the skin.

According to another aspect of the present disclosure, a system for selecting a paint composition includes a user input interface configured to receive components and amounts of candidate raw materials of a paint composition, and a controller configured to verify hypersensitivity component information of the candidate raw materials, calculate a skin hypersensitivity index of the paint based on the components and amounts of the candidate raw materials and the

hypersensitivity component information, evaluate the harmfulness of the paint to the skin based on the skin hypersensitivity index of the paint, and select a paint composition based on the evaluated harmfulness of the paint to the skin.

## Advantageous Effects of Invention

According to the present disclosure, it is possible to quantify a skin hypersensitivity index of paint based on hypersensitivity information, corrosiveness information, and the amount of substances in components of the paint, and to objectively and systematically evaluate harmfulness of paint to skin based on the skin hypersensitivity index.

In addition, according to an embodiment of the present disclosure, by calculating hypersensitivity indices of a base material and a curing agent separately and then calculating a final hypersensitivity index of paint based on a mixing ratio of the base material and the curing agent, it is possible to easily select the final paint formulation.

In addition, by objectively and systematically evaluating the harmfulness of the paint, it is possible to develop paint formulations with low skin irritation. Accordingly, it is possible to develop paint that ensures worker safety and to implement safe working environments.

In addition, the effects obtainable or predicted by the embodiments of the present disclosure will be disclosed directly or implicitly in the detailed description of the embodiments of the present disclosure. That is, various effects predicted according to embodiments of the present disclosure will be disclosed in the detailed description to be described later.

## Brief Description of Drawings

Embodiments of the present specification can be better understood with reference to the accompanying drawings, in which like reference numerals refer to the same or functionally similar elements.

FIG. 1 is a block diagram of a system for selecting a paint composition according to an embodiment of the present disclosure.

FIG. 2 is a flowchart of a method for selecting a paint composition according to an embodiment of the present disclosure.

FIG. 3 is a flowchart of the method for selecting a paint composition according to an embodiment of the present disclosure.

FIG. 4 is a flowchart of step S220 of FIG. 3.

FIG. 5 is a flowchart of step S230 of FIG. 3.

FIG. 6 is a diagram illustrating an example of a hypersensitivity calculation table.

FIG. 7 is a diagram illustrating an example of a database storing hypersensitivity grades and corrosiveness grades of raw materials constituting paint. It should be understood that the drawings referenced above are not necessarily drawn to scale, and present rather simplified representations of various preferred features illustrating the basic principles of the present disclosure. For example, specific design features of the present disclosure, including specific dimensions, direction, position, and shape, will be determined in part by specific intended applications and use environments.

## Best Mode for the Invention

The terminology used herein is for the purpose of describing particular embodiments only, and is not intended to limit the present disclosure. As used herein, singular forms are intended to also include plural forms, unless the context clearly dictates otherwise. The terms "comprises" and/or "comprising," specify the cited features, integers, steps, operations, elements, and/or the presence of components when used herein, but it will also be understood that these terms do not exclude the presence or addition of one or more of other features, integers, steps, operations, elements, components, and/or groups thereof. As used herein, the term "and/or" includes any one or all combinations of the associated listed items.

Additionally, it is understood that one or more of the methods below or aspects thereof may be executed by at least one or more controllers. The term "controller" may refer to a hardware device including a memory and a processor. The memory is configured to store program instructions, and the processor is specifically programmed to execute the program instructions to perform one or more processes described in more detail below. A controller may control operations of units, modules, parts, devices, or the like, as described herein. It is also understood that methods below may be executed by an apparatus including a controller in conjunction with one or more other components, as will be appreciated by those skilled in the art.

Additionally, a controller of the present disclosure may be implemented as a non-transitory computer-readable recording medium including executable program instructions executed by a processor. Examples of computer-readable

recording media include, but are not limited to, read-only memory (ROM), random-access memory (RAM), compact disc (CD) ROMs, magnetic tapes, floppy disks, flash drives, smart cards, and optical data storage devices. The computer-readable recording medium may also be distributed across a computer network, such that the program instructions may be stored and executed in a distributed manner, such as via a telematics server or a controller area network (CAN).

According to an embodiment of the present disclosure, by inputting raw materials constituting a base material and a curing agent of paint, the amount of raw materials, and a mixing ratio of the base material and the curing agent, a skin hypersensitivity index is calculated, and a paint's risk grade may be automatically calculated based on a skin hypersensitivity index. In addition, by objectively and systematically evaluating the harmfulness of the paint, it is possible to develop paint formulations with low skin irritation and support selecting the paint composition.

In this specification, "skin harmfulness" may include a meaning of skin hypersensitivity, skin corrosion, and/or skin irritation. A skin hypersensitivity substance refers to a substance that causes skin allergies upon contact with the skin, a skin corrosiveness substance refers to a substance that causes irreversible skin damage, and a skin irritation substance refers to a substance that causes reversible skin damage. In this specification, the "hypersensitivity" refers to skin hypersensitivity, and the "corrosion" refers to skin corrosion.

When "paint" is used herein, the paint may include the base material and the curing agent.

Furthermore, unless otherwise specified, the units of "amount" used in this specification are based on weight. For example, "%" or "ratio" refers to a weight % or a weight ratio.

Hereinafter, embodiments of the present disclosure will be described in detail with reference to the accompanying drawings.

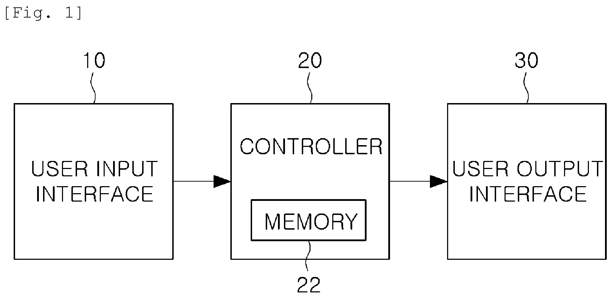

FIG. 1 is a block diagram of a system for evaluating harmfulness of paint according to an embodiment of the present disclosure.

As illustrated in FIG. 1, the system for evaluating harmfulness of paint according to an embodiment of the present disclosure includes a user input interface 10, a controller 20, and a user output interface 30. The user input interface 10, the controller 20, and the user output interface 30 are connected to each other wirelessly or wiredly to exchange data.

The user input interface 10 may be configured to receive, from a user, the raw materials constituting the base material and the curing agent of paint, the amount of raw materials, and/or a mixing ratio of the base material and the curing agent. For example, the user input interface 10 may include a keyboard, a mouse, a touch screen, a microphone, a joystick, a jog shuttle, a stylus, etc. Furthermore, the user input interface 10 may include soft keys implemented on a display.

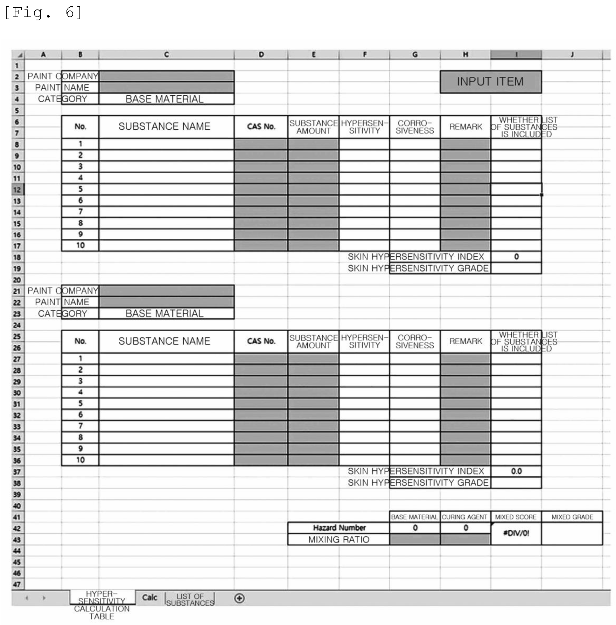

In one example, a hypersensitivity calculation table as illustrated in FIG. 6 may be displayed on a user output interface 30 so that a user may receive the raw materials constituting the base material and the curing agent of the paint, the amount of raw materials, and/or the mixing ratio of the base material and the curing agent through the user input interface 10. That is, the user inputs material names (e.g., CAS No.) of the raw materials constituting the base material and the amount of each raw material on the hypersensitivity calculation table, the material names (e.g., CAS No.) of the raw materials constituting the curing agent, and the amount of each raw material. In addition, the user inputs the mixing ratio of the base material and the curing agent on the hypersensitivity calculation table.

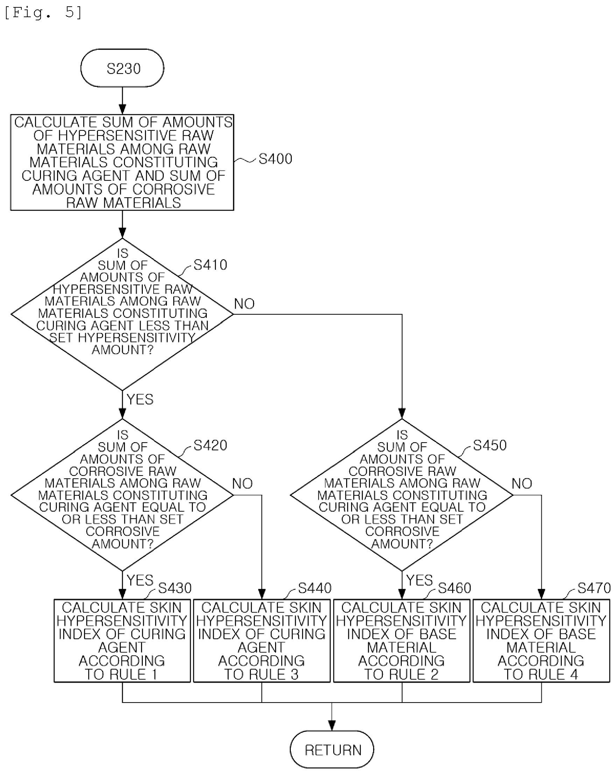

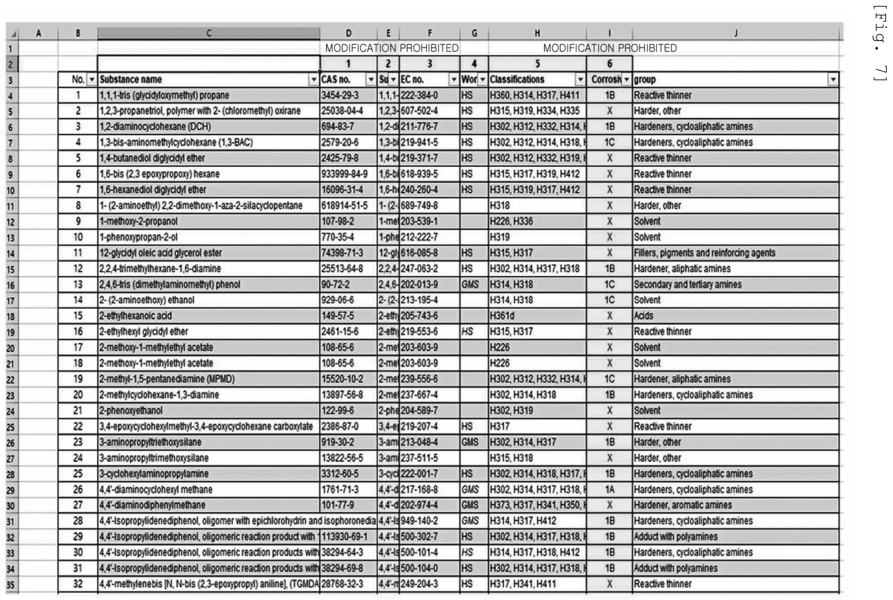

The controller 20 obtains hypersensitivity component information and/or corrosiveness component information of the raw materials constituting the base material and the curing agent based on the material names (e.g., CAS No.) of the raw materials constituting the base material and the curing agent input through the user input interface 10. That is, when a user inputs the material names of the raw materials constituting the base material and the curing agent into the hypersensitivity calculation table, as illustrated in FIG. 7, the controller 20 may read the hypersensitivity component information and/or the corrosiveness component information of each raw material from the database of the raw materials. The hypersensitivity component information may include at least one of information about whether the corresponding component corresponds to a hypersensitivity component and information about a hypersensitivity grade, and the corrosiveness component information may include at least one of information about whether the corresponding component corresponds to the corrosiveness component and information about the corrosiveness grade. To this end, the controller 20 includes a memory 22, and the memory 22 stores a hypersensitivity calculation table as illustrated in FIG. 5 and a database of raw materials as illustrated in FIG. 7. The hypersensitivity calculation table as illustrated in FIG. 5 and the database of raw materials as illustrated in FIG. 7 are merely examples, and the hypersensitivity calculation table and the database of raw materials are not limited to those illustrated in FIG. 6 and FIG. 7, respectively.

Referring to FIG. 7, the database of the raw materials stores the hypersensitivity grade and corrosiveness grade of raw materials used in the base material and curing agent of the paint. For example, the hypersensitivity grade of raw materials is classified as 'HS' and 'GMS', where 'HS' indicates 'high hypersensitivity', and 'GMS' indicates 'low or normal hypersensitivity.' Furthermore, when nothing is entered in a hypersensitivity grade cell of raw materials, it means that the raw materials are not a hypersensitivity substance. Furthermore, the corrosiveness grades of raw materials are classified as '1A', '1B', and '1C', where '1A' indicates 'high corrosiveness', '1B' indicates normal corrosiveness' and '1C' indicates 'low corrosiveness'. Furthermore, a raw material marked with 'X' in the corrosiveness grade cell indicates that the raw material is not a corrosive substance.

The database may be an Epoxy Information System (EIS), a Material Safety Data Sheet (MSDS), the

Classification, Labeling, and Packaging Regulations of Chemical Substances and Mixtures to the Globally Harmonized System (GHS) (CLP Regulation), the Korea Occupational Safety and Health Agency (KOSHA), or the European Chemicals Agency (ECHA). The databases may be integrated or supplemented. For example, when it is difficult to accurately determine the hypersensitivity grade and/or corrosiveness grade of raw materials using only a specific database, the specific database may be supplemented using data from other databases. Furthermore, missing information in a specific database may be supplemented through additional experiments.

The controller 20 calculates the skin hypersensitivity indices of the base material and the curing agent according to the established rules based on the amount of raw materials constituting the base material and the curing agent of the paint input through the user input interface 10 and the hypersensitivity grade and/or corrosiveness grade of the raw materials retrieved from the database. Among the base material and the curing agent, the curing agent generally has the higher skin hypersensitivity than the base material, but the amount of the curing agent in paint is lower. When the skin hypersensitivity index of the paint is determined as a whole without considering the skin hypersensitivity indices of the base material and the curing agent separately, the skin hypersensitivity of the curing agent with the relatively low amount and high skin hypersensitivity may not be revealed in the skin hypersensitivity index of the paint. Therefore, to select an appropriate base material or curing agent during the development of the paint, the skin hypersensitivity indices of the base material and the curing agent are derived separately, and then the skin hypersensitivity index of the paint is calculated using the skin hypersensitivity indices of the base material and the curing agent.

To calculate the skin hypersensitivity indices of the base material and the curing agent, the memory 22 of the controller 20 may store the established rules. As an example, (Table 1) shows rules for calculating the skin hypersensitivity indices of the base material and the curing agent. However, the rules for calculating the skin hypersensitivity indices of the base material and the curing agent are not limited to those shown in (Table 1).

[Table 1]

| Category | Application conditions | Calculation method |
|---|---|---|
| Rule 1 | Sum of corrosiveness components ≤ sum of 10%hypersensitivity component < 40% | - HS: increases by 15 points per 1% - GMS: increases by 5 points per 1% |
| Rule 2 | sum of corrosiveness components ≤ sum of 10%hypersensitivity component ≥ 40% | - Rule 1 is applied until the sum of hypersensitivity components reaches 40% (priority is given to HS) - For the portion by which the sum of hypersensitivity components exceeds 40%, HS: increases by 2.5 points per 1%, and GMS: increases by 2.5 points per 1% - When the sum of hypersensitivity components exceeds 40%, priority is given to GMS. |
| Rule 3 | Sum of corrosiveness components > sum of 10%hypersensitivity components < 40% | - Rule 1 is applied to a skin hypersensitivity index without considering corrosiveness - A skin hypersensitivity index is calculated as a product of a skin hypersensitivity index without considering corrosiveness and a weight based on corrosiveness components - A weight based on corrosiveness components is applied starting from when the sum of corrosiveness components exceeds 10% - A weight assigned according to a corrosiveness grade of a corrosive substance is as follows: 1A: 0.2, 1B: 0.15, and 1C: 0.1. |
| Rule 4 | Sum of corrosiveness components > sum of 10%hypersensitivity component ≥ 40% | - Rule 2 is applied to a skin hypersensitivity index without considering corrosiveness - A skin hypersensitivity index is calculated as a product of a skin hypersensitivity index without considering corrosiveness and a weight based on corrosiveness components - A weight based on corrosiveness components is applied starting from when the sum of corrosiveness components exceeds 10% - A weight assigned according to a corrosiveness grade of a corrosive substance is as follows: 1A: 0.2, 1B: 0.15, and 1C: 0.1. |

According to the report "OPINION ON COMPARATIVE HEALTH EVALUATION OF EPOXY RESIN SYSTEMS BELOW CONSIDERATION OF Awareness-raising EFICIENCY (FP-0384)" published by the German Social Accident Insurance (DGUV) in December 2016, in a mixture, the hypersensitivity effect of the hypersensitivity component increases linearly up to a maximum proportion of 40%, with the influence factors varying according to the hypersensitivity grade (HS, GMS). The influence factor may mean the degree to which the hypersensitivity component influences the hypersensitivity

effect. For example, the influence factor of a high hypersensitivity (HS) component may be greater than that of a low hypersensitivity (GMS) component. Furthermore, when the ratio of the hypersensitivity component exceeds 40%, the influence factor value may decrease, and it may be assumed that the influence factors of the high hypersensitivity (HS) component and the low hypersensitivity (GMS) component are equal.

Furthermore, according to the report, when the mixture contains a corrosiveness component equal to or less than 10%, the corrosiveness effect may be insignificant, and when the mixture contains a corrosiveness component equal to or greater than 10%, the corrosiveness effect may be observed.

Therefore, when calculating the skin hypersensitivity index for assessing the hazard level of paint, different rules may be applied depending on whether the ratio of the hypersensitivity component in the mixture exceeds a certain reference (e.g., 40%) or the ratio of the corrosiveness component exceeds a certain reference (e.g., 10%), thereby calculating the skin hypersensitivity index.

Furthermore, the corrosiveness component may damage a skin barrier to facilitate the penetration of the hypersensitivity substances into the skin, thereby inducing the high skin hypersensitivity. Therefore, when the ratio of the corrosiveness components exceeds a certain reference (e.g., 10%) and exhibits the corrosiveness effect, a weight may be assigned to the hypersensitivity index.

As illustrated in Table 1, the rules for calculating the skin hypersensitivity indices of the base material and the curing agent include four rules, Rules 1 through 4. Rule 1 is applied when, among the raw materials constituting the base material or the curing agent, the sum of the corrosiveness components is equal to or less than 10% and the sum of the hypersensitivity components is less than 40%. According to Rule 1, 15 points are assigned per 1% of high hypersensitivity (HS) components, and 5 points are assigned per 1% of low hypersensitivity (GMS) components, and the skin hypersensitivity indices for all hypersensitivity components are summed.

Rule 2 is applied when, among the raw materials constituting the base material or the curing agent, the sum of the corrosiveness components is equal to or less than 10% and the sum of the hypersensitivity components is equal to or greater than 40%. According to Rule 2, Rule 1 is applied until the sum of the hypersensitivity components reaches 40%. As the amount of skin hypersensitivity substance increases, the frequency of skin hypersensitivity increases linearly with its amount, and then, when the sum of the total skin hypersensitivity components is equal to or greater than 40%, the increase in the frequency of skin hypersensitivity decreases. Therefore, Rule 1 is applied until the sum of the hypersensitivity components reaches 40%. In this case, priority is given to the high hypersensitivity (HS) components. That is, until the sum of the hypersensitivity components reaches 40%, 15 points are first assigned per 1% of high hypersensitivity (HS) components, and then 5 points are assigned per 1% of low hypersensitivity (GMS) components.

Thereafter, for the components in which the total hypersensitivity components exceed 40%, 2.5 points are assigned per 1% for both the high hypersensitivity (HS) component and the low hypersensitivity (GMS) component. When the amount of total hypersensitivity components exceeds 40%, the increase amount in the frequency of skin hypersensitivity decreases, but the high skin hypersensitivity substances are more likely to cause skin hypersensitivity reactions than the low skin hypersensitivity substances. Therefore, when both the high and low skin hypersensitivity substances are contained in the base material or the curing agent, priority is given to the high skin hypersensitivity substances until the total hypersensitivity components reach 40%, and priority is given to the low skin hypersensitivity substances for the amount in which the sum of the hypersensitivity components exceeds 40%, thereby preventing the overall skin hypersensitivity index of the base material or the curing agent from being lowered by the low skin hypersensitivity substance.

For example, when the components of raw materials constituting the base material or the curing agent contain 30% of the high hypersensitivity (HS) components and 20% of the low hypersensitivity (GMS) components, 15 points are first assigned to 30% of the high hypersensitivity component, 5 points are assigned to 10% of the low hypersensitivity component (i.e., 40% to 30%), and 2.5 points are assigned to the remaining 10% of low hypersensitivity components (20% to 10%), so the hypersensitivity index becomes 525 (= 30*15 + 10*5 + 10*2.5).

Rule 3 is applied when, among the raw materials constituting the base material or the curing agent, the sum of the corrosiveness components exceeds 10% and the sum of the hypersensitivity components is less than 40%. The components indicating the skin corrosiveness act by corroding the skin barrier, thereby increasing the penetration of the skin hypersensitivity substances. Therefore, when the base material or the curing agent contains the skin corrosiveness component along with the skin hypersensitivity component, in calculating the skin hypersensitivity index, the skin corrosiveness component may be applied as a weight. However, since the skin corrosiveness components may corrode the skin barrier when present at a concentration of 10% or more, the weight based on the skin corrosiveness component may only be applied when the component exhibiting the skin corrosiveness is contained 10% or more.

According to Rule 3, the skin hypersensitivity index without considering corrosiveness is calculated according to Rule 1, and the skin hypersensitivity index is calculated by multiplying the skin hypersensitivity index without considering the corrosiveness by the weight based on the corrosiveness component. Here, the weight based on the corrosiveness component is assigned when the sum of the corrosiveness components exceeds 10%. The weight based on the grade of the corrosive substance is 0.2 per 1% of the high-corrosiveness (1A) component, 0.15 per 1% of the medium-corrosiveness (1B) component, and 0.1 per 1% of the low-corrosiveness (1C) component.

For example, (Table 2) shows the amount, hypersensitivity information, and corrosiveness information of the base material composed of three substances (substance A, substance B, and substance C). In (Table 2), the sum of the hypersensitivity components of three substances is 15%, and the sum of the corrosiveness components is 30%, so Rule 3 is applied.

[Table 2]

| Category | Amount | Hypersensitivity information | Corrosiveness information |
|---|---|---|---|
| Material A | 5% | HS | 1A |
| Material B | 10% | GMS | 1B |
| Material C | 15% | X | 1C |

First, the skin hypersensitivity index without considering corrosiveness is 125 that is the sum of the skin hypersensitivity index (5*15=75) of the substance A and the skin hypersensitivity index (10*5=50) of the substance B. Furthermore, the weight based on the corrosiveness component is 4, which is the sum of the weight (5*0.2=1) of the substance A, the weight (10*0.15=1.5) of the substance B, and the weight (15*0.1=1.5) of the substance C. Therefore, the skin hypersensitivity index of the base material shown in Table 2 is calculated as 125*4=500.

Rule 4 is applied when, among the raw materials constituting the base material or the curing agent, the sum of the corrosiveness components exceeds 10% and the sum of the hypersensitivity components is equal to or greater than 40%. According to Rule 4, since the sum of the hypersensitivity components is equal to or greater than 40%, the skin hypersensitivity index without considering the corrosiveness is calculated according to Rule 2, and the skin hypersensitivity index is calculated by multiplying the skin hypersensitivity index without considering the corrosiveness by the weight based on the corrosiveness component. Here, the weight based on the corrosiveness component is assigned when the sum of the corrosiveness components exceeds 10%. The weight based on the grade of the corrosive substance is 0.2 per 1% of the high-corrosiveness (1A) component, 0.15 per 1% of the medium-corrosiveness (1B) component, and 0.1 per 1% of the low-corrosiveness (1C) component.

The controller 20 calculates the skin hypersensitivity index of the paint based on the skin hypersensitivity indices of the base material and the curing agent calculated above and the mixing ratio of the base material and the curing agent input through the user input interface 10. For example, the skin hypersensitivity index of the paint may be calculated using the following equation (Equation 1):

Hypersensitivity index of paint = hypersensitivity index of base material * mixing fraction of base material + hypersensitivity index of curing agent * mixing fraction of curing agent (Equation 1)

The controller 20 may evaluate the harmfulness of the paint based on whether the calculated skin hypersensitivity index of the paint is equal to or greater than the reference hypersensitivity index.

For example, when the reference hypersensitivity index is set to 1,500 points, the controller 20 may determine that the paint is harmful when the calculated skin hypersensitivity index of the paint is equal to or greater than 1,500 points, and determine that the paint is harmless or non-harmful when the calculated skin hypersensitivity index of the paint is less than 1,500 points. Here, the reference hypersensitivity index may be set differently depending on the scoring method or experiment.

When scores are assigned based on Table 1 of this specification and based on animal experiments or alternative animal tests described below, the reference hypersensitivity index may be set at 1200 to 1700 points, and more preferably around 1500 points. In this case, the animal test may preferably be performed using the OECD TG 404 test for skin irritation/corrosiveness and the OECD TG 406 test for skin hypersensitivity. The alternative animal test may preferably be performed using the OECD TG 435 test for skin corrosiveness and the OECD TG 442B test for skin hypersensitivity.

Here, the reference hypersensitivity index may be established through the animal or alternative animal test. For example, by checking a result of assessing the harmfulness in the animal or alternative animal test based on the calculated skin hypersensitivity index of the paint, when the hypersensitivity index exceeds a certain reference and the probability that the paint will be assessed to be harmful in the animal or alternative animal test increases, the certain hypersensitivity index may be set as the reference hypersensitivity index. According to an embodiment of the present disclosure, by setting the reference hypersensitivity index, the harmfulness of the paint may be easily predicted by inputting the components and amounts of base material and the curing agent and the mixing ratio.

In another embodiment, the controller 20 may divide the calculated skin hypersensitivity index of the paint into the set risk grades. For example, as shown in (Table 3), the controller 20 may divide the skin hypersensitivity index into 11 risk grades in units of 200 points. However, it should be understood that the set risk grades are not limited to 11 stages.

[Table 3]

| Skin hypersensitivity index | Risk grade |
|---|---|
| ~200 | I |
| 200~400 | II |
| 400~600 | III |
| 600~800 | IV |
| 800~1000 | V |
| 1000~1200 | VI |
| 1200~1400 | VII |
| 1400~1600 | VIII |
| 1600~1800 | IX |
| 1800~2000 | X |
| 2000~ | X I |

In Table 3, a higher risk grade indicates that the substance is evaluated as being more harmful to the skin. Thus, according to an embodiment of the present disclosure, the skin hypersensitivity of the paint may be quantified using the skin hypersensitivity index, thereby objectively and systematically evaluating the risk grade. When a new paint is developed, the risk grade of the new paint may be calculated by entering the raw materials constituting the base material and the curing agent of the paint into the hypersensitivity calculation table. Therefore, it is possible to support the development of paints with lower risk grades.

For this purpose, the controller 20 may be implemented as one or more processors operated by a set program, and the memory 22 of the controller 20 stores program instructions programmed to perform each step of the method for selecting a paint composition according to an embodiment of the present disclosure via the one or more processors.

The user output interface 30 displays the hypersensitivity calculation table as illustrated in FIG. 6, allowing the user to input the raw materials constituting the base material and the curing agent of the paint, their amounts, and/or the mixing ratio of the base material and the curing agent through the user input interface 10. Furthermore, the user output interface 30 may display the results of executing the program set by the controller 20 or issue a warning using sound, etc. For example, the user output interface 30 may display the skin hypersensitivity indices of the base material and the curing agent, the skin hypersensitivity index of the paint, and/or the risk grade calculated by the controller 20. Furthermore, the user output interface 30 may issue a warning using a screen, sound, etc., when the risk grade is high.

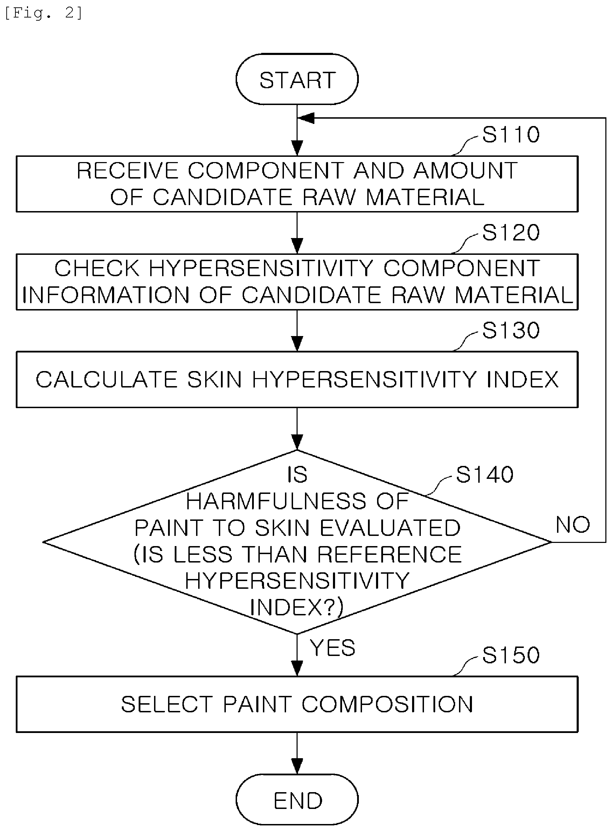

FIG. 2 is a flowchart of a method for selecting a paint composition according to an embodiment of the present disclosure.

Referring to FIG. 2, the components and amounts of the candidate raw materials of the paint composition may be received through the user input interface (S110). A paint composition may be composed of a mixture of the base material and the curing agent. The hypersensitivity component information of the candidate raw materials may be checked (S120). The hypersensitivity component information of the candidate raw materials may be stored in the database. The hypersensitivity component information may include whether the component of the candidate raw materials corresponds to the hypersensitivity component and the information about the hypersensitivity grade. The hypersensitivity grade may include a high hypersensitivity grade and a low or normal hypersensitivity grade.

Then, based on the components and amounts of the candidate raw materials and the hypersensitivity component information, the skin hypersensitivity index of the paint may be calculated (S130).

The harmfulness of the paint to the skin may be evaluated based on the skin hypersensitivity index (S140). Furthermore, the paint composition may be selected based on the evaluated harmfulness of paint of the skin (S150).

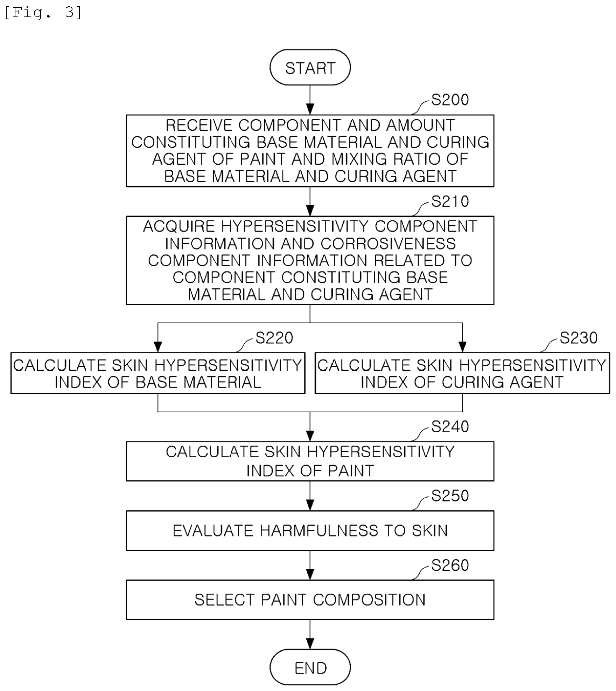

Hereinafter, the method for selecting a paint composition according to an embodiment of the present disclosure will be described in more detail with reference to FIGS. 3 to 5.

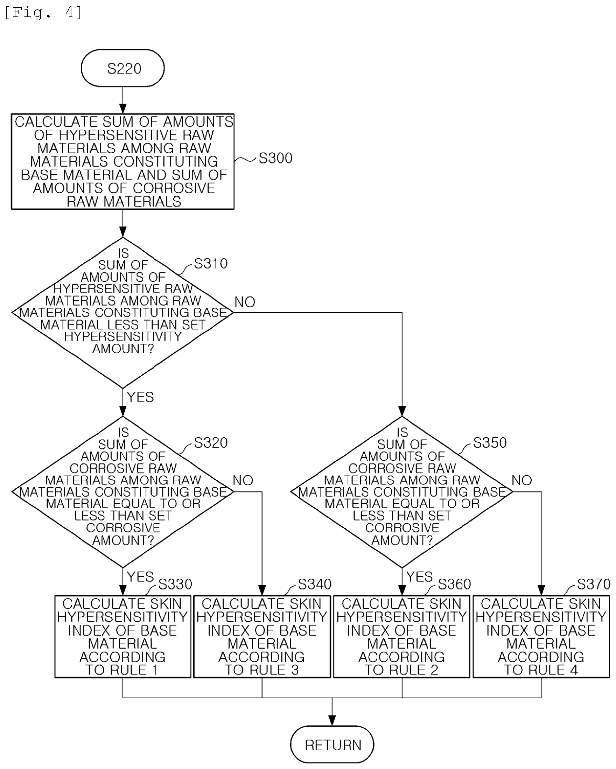

FIG. 3 is a flowchart of the method for selecting a paint composition according to an embodiment of the present disclosure, FIG. 4 is a flowchart of step S220 of FIG. 3 , and FIG. 5 is a flowchart of step S230 of FIG. 3.

As illustrated in FIG. 3, the method for selecting a paint composition according to an embodiment of the present disclosure receives the components of raw materials constituting the base material and the curing agent of the paint, their amounts, and the mixing ratio of the base material and the curing agent (S200). For example, the controller 20 may display the hypersensitivity calculation table as illustrated in FIG. 6 through the user output interface 30 so that a user may input the raw materials constituting the base material and the curing agent of the paint, their amounts, and the mixing ratio of the

base material and the curing agent through the user input interface 10, and the controller 20 may receive the raw materials constituting the base material and the curing agent of the paint, their amounts, and the mixing ratio of the base material and the curing agent input by the user.

The controller 20 recognizes the raw materials constituting the base material and the curing agent of the paint input by the user and acquires the hypersensitivity component information and/or the corrosiveness component information of the raw materials constituting the base material and the curing agent of the paint (S210). The hypersensitivity component information may include at least one of information about whether the corresponding component corresponds to a hypersensitivity component and information about a hypersensitivity grade, and the corrosiveness component information may include at least one of information about whether the corresponding component corresponds to the corrosiveness component and information about the corrosiveness grade. For example, the controller 20 may retrieve the hypersensitivity component information and/or the corrosiveness component information of the raw materials constituting the base material and the curing agent of the paint from the database stored in the memory 22.

When the hypersensitivity grade and/or the corrosiveness grade of the raw materials constituting the base material or the curing agent are acquired, the controller 20 calculates the skin hypersensitivity of the base material according to the established rules (S220). Step S220 will be described in more detail with reference to FIG. 4.

As illustrated in FIG. 4, the controller 20 calculates the sum of the amounts of hypersensitive raw materials and the sum of the amounts of corrosive raw materials among the raw materials constituting the base material to determine the rule to be applied for calculating the skin hypersensitivity of the base material (S300). That is, since one of Rules 1 to 4 is applied depending on the sum of the amounts of hypersensitive raw materials and the sum of the amounts of corrosive raw materials, the controller 20 calculates the sum of the amounts of hypersensitive raw materials and the sum of the amounts of corrosive raw materials among the raw materials constituting the base material.

Thereafter, the controller 20 determines whether the amount of hypersensitive raw materials among the raw materials constituting the base material is less than a set hypersensitivity amount (e.g., 40%) (S310). In step S310, when it is determined that the amount of hypersensitive raw materials among the raw materials constituting the base material is less than the set hypersensitivity amount (e.g., 40%), the controller 20 determines whether the sum of the amounts of corrosive raw materials among the raw materials constituting the base material is equal to or less than the set corrosive amount (e.g., 10%) (S320).

In step S320, when the sum of the amounts of corrosive raw materials among the raw materials constituting the base material is equal to or less than the set corrosive amount (e.g., 10%), the controller 20 calculates the skin hypersensitivity index of the base material according to Rule 1 (S330). For example, 15 points are assigned for each 1% of high hypersensitivity (HS) components, and 5 points are assigned for each 1% of low hypersensitivity (GMS) components, and the skin hypersensitivity indices for all hypersensitivity components are summed.

In step S320, when the sum of the amounts of corrosive raw materials among the raw materials constituting the base material exceeds the set corrosive amount (e.g., 10%), the controller 20 calculates the skin hypersensitivity index of the base material according to Rule 3 (S340). For example, the controller 20 calculates the skin hypersensitivity index as the product of the skin hypersensitivity index without considering the corrosiveness and the weight based on the corrosiveness component. The skin hypersensitivity index without considering the corrosiveness is calculated according to Rule 1, the weight based on the corrosiveness component is assigned when the sum of corrosiveness components exceeds 10%, and the weight assigned according to the grade of the corrosive substance is 0.2 per 1% of the high corrosiveness (1A) component, 0.15 per 1% of the normal corrosiveness (1B) component, and 0.1 per 1% of the low corrosiveness (1C) component.

In step S310, when it is determined that the amount of hypersensitive raw materials among the raw materials constituting the base material is equal to or greater than the set hypersensitivity amount (e.g., 40%), the controller 20 determines whether the sum of the amounts of corrosive raw materials among the raw materials constituting the base material is equal to or less than the set corrosive amount (e.g., 10%) (S350).

In step S350, when the sum of the amounts of corrosive raw materials among the raw materials constituting the base material is equal to or less than the set corrosive amount (e.g., 10%), the controller 20 calculates the skin hypersensitivity index of the base material according to Rule 2 (S360). For example, Rule 1 is applied until the sum of the hypersensitivity components reaches 40%, but priority is given to the high skin hypersensitivity substances, and for the components in which the sum of the hypersensitivity components exceeds 40%, 2.5 points are assigned per 1% for both the high hypersensitivity (HS) components and low hypersensitivity (GMS) components.

In step S350, when the sum of the amounts of corrosive raw materials among the raw materials constituting the base material exceeds the set corrosive amount (e.g., 10%), the controller 20 calculates the skin hypersensitivity index of the base material according to Rule 4 (S370). For example, the controller 20 calculates the skin hypersensitivity index as the product of the skin hypersensitivity index without considering the corrosiveness and the weight based on the corrosiveness component. The skin hypersensitivity index without considering the corrosiveness is calculated according to Rule 2, the weight based on the corrosiveness component is assigned when the sum of corrosiveness components exceeds 10%, and the weight assigned according to the grade of the corrosive substance is 0.2 per 1% of the high

corrosiveness (1A) component, 0.15 per 1% of the normal corrosiveness (1B) component, and 0.1 per 1% of the low corrosiveness (1C) component.

In addition, the controller 20 calculates the skin hypersensitivity of the curing agent according to the set rules (S230). Step S230 will be described in more detail with reference to FIG. 5.

As illustrated in FIG. 5, the controller 20 calculates the sum of the amounts of hypersensitive raw materials and the sum of the amounts of corrosive raw materials among the raw materials constituting the curing agent to determine the rule to be applied for calculating the skin hypersensitivity of the curing agent (S400).

Thereafter, the controller 20 determines whether the amount of hypersensitive raw materials among the raw materials constituting the curing agent is less than a set hypersensitivity amount (e.g., 40%) (S410). In step S410, when it is determined that the amount of hypersensitive raw materials among the raw materials constituting the curing agent is less than the set hypersensitivity amount (e.g., 40%), the controller 20 determines whether the sum of the amounts of corrosive raw materials among the raw materials constituting the curing agent is equal to or less than the set corrosive amount (e.g., 10%) (S420).

In step S420, when the sum of the amounts of corrosive raw materials among the raw materials constituting the curing agent is equal to or less than the set corrosive amount (e.g., 10%), the controller 20 calculates the skin hypersensitivity index of the curing agent according to Rule 1 (S430), and in step S420, the sum of the amounts of corrosive raw materials among the raw materials constituting the curing agent exceeds the set corrosive amount (e.g., 10%), the controller 20 calculates the skin hypersensitivity index of the curing agent according to Rule 3 (S440).

In step S410, when it is determined that the amount of hypersensitive raw materials among the raw materials constituting the curing agent is equal to or greater than the set hypersensitivity amount (e.g., 40%), the controller 20 determines whether the sum of the amounts of corrosive raw materials among the raw materials constituting the curing agent is equal to or less than the set corrosive amount (e.g., 10%) (S450).

In step S450, when the sum of the amounts of corrosive raw materials among the raw materials constituting the curing agent is equal to or less than the set corrosive amount (e.g., 10%), the controller 20 calculates the skin hypersensitivity index of the curing agent according to Rule 2 (S460), and in step S450, the sum of the amounts of corrosive raw materials among the raw materials constituting the curing agent exceeds the set corrosive amount (e.g., 10%), the controller 20 calculates the skin hypersensitivity index of the curing agent according to Rule 4 (S470).

Referring back to FIG. 3, when the skin hypersensitivity index of the base material is calculated in step S220 and the skin hypersensitivity index of the curing agent is calculated in step S230, the controller 20 calculates the skin hypersensitivity index of the paint based on the skin hypersensitivity indices of the base material and the curing agent and the mixing ratio of the base material and the curing agent (S240). For example, the controller 20 may calculate the skin hypersensitivity index of the paint using Equation 1.

Thereafter, the controller 20 evaluates the calculated harmfulness of the paint to the skin based on the skin hypersensitivity index (S250). For example, when the calculated skin hypersensitivity index of the paint is less than the reference hypersensitivity index, the controller 20 may determine that the paint is harmless, and when the calculated skin hypersensitivity index is equal to or greater than the reference hypersensitivity index, the controller 20 may determine that the paint is harmful.

For example, when the reference hypersensitivity index is set to 1,500 points, the controller 20 may determine that the paint is harmful when the calculated skin hypersensitivity index of the paint is equal to or greater than 1,500 points, and determine that the paint is harmless or non-harmful when the calculated skin hypersensitivity index of the paint is less than 1,500 points. Alternatively, as shown in Table 3, the controller 20 may classify the calculated skin hypersensitivity index of the paint to the skin as one of 11 risk grades and determine that the paint is harmful when the calculated skin hypersensitivity index is equal to or greater than the set risk grade.

The controller 20 may select the paint composition based on the evaluated skin harmfulness assessment (S260). When, as a result of evaluating skin harmfulness, the calculated skin hypersensitivity index is equal to or greater than the reference hypersensitivity index and thus the paint is evaluated as harmful, the paint may not be selected as part of the paint composition. Subsequently, the components, amounts, and mixing ratio of the new composition are reentered through the user input interface 10, and the new composition may be evaluated.

Alternatively, the controller 20 may select the paint composition when the skin hypersensitivity index calculated as a result of evaluating the harmfulness to the skin is less than the reference hypersensitivity.

According to an embodiment of the present disclosure, by providing objective criteria and methods for evaluating paint compositions, it is possible to select a paint composition with low skin harmfulness.

Additionally, the controller 20 may display the skin hypersensitivity index of the base material and the curing agent, the skin hypersensitivity index of the paint, and/or the risk grade through the user output interface 30. When the risk grade is high, the controller 20 may issue a warning through a screen, sound, etc., through the user output interface 30.

While steps S220 and S230 are described as being performed sequentially in this specification, the order of steps S220 and S230 may differ from the disclosed order. Specifically, step S220 may be performed after step S230.

Example 1

Hereinafter, the process for calculating the skin hypersensitivity index and risk grade of the paint according to Example 1 is described in more detail. (Table 4) shows the raw materials constituting the base material and the curing agent of the paint according to Example 1, along with their amounts and the mixing ratio.

[Table 4]

| Base material | | | | Curing agent | | | |
|---|---|---|---|---|---|---|---|
| Constituent component | Amount( %) | Hypersensitivity | Corrosiveness | Constituent component | Amount( %) | Hypersensitivity | Corrosiveness |
| epoxy-formal dehyde resin | 50 | HS | X | Carbomonocyclic alkylated | 65 | HS | 1B |
| Silane , trimet hoxy | 10 | GMS | X | Formal dehyde , oligomeric reaction | 10 | HS | X |
| oxirane, 2,2'-[1,6-hex-ane diylbis | 10 | HS | X | 2,6-ditert - butyl-p-cresol | 10 | HS | X |
| | | | | 3-aminopropyldiethyl amine | 5 | HS | 1B |
| Hypersensitivity index | | 675 | | Hypersensitivity index | | 7612.5 | |
| Mixing ratio= 4: 1, skin hypersensitivity index = 2062.5 (risk grade X I ) | | | | | | | |

First, the controller 20 displays the corrosiveness calculation table, as shown in Table 4, through the user output interface 30. The user enters the raw materials constituting the base material and the curing agent of the paint, their amounts, and the mixing ratio of the base material and the curing agent through the user input interface 10.

The controller 20 retrieves the hypersensitivity grade and the corrosiveness grade of each raw material from the database stored in memory 22 and calculates the skin hypersensitivity indices of the base material and the curing agent.

To calculate the skin hypersensitivity index of the base material, the sum of the amount of hypersensitive raw material and the sum of the amount of corrosive raw material among the raw materials constituting the base material are calculated to be 70% and 0%, respectively. Accordingly, the skin hypersensitivity index of the base material is calculated as follows according to Rule 2.

Skin hypersensitivity index of base material = 40*15+10*2.5+10*2.5+10*2.5 = 675

To calculate the skin hypersensitivity index of the curing agent, the sum of the amount of hypersensitive raw material and the sum of the amount of corrosive raw material among the raw materials constituting the curing agent are calculated to be 90% and 70%, respectively. Accordingly, the skin hypersensitivity index of the base material is calculated as follows according to Rule 4.

Hypersensitivity index without considering corrosiveness = 40*15 + 25*2.5 + 10*2.5 + 5*2.5 = 725

Weight based on corrosiveness = 65*0.15 + 5*0.15 = 10.5

Skin hypersensitivity index of curing agent = 725*10.5 = 7612.5

The controller 20 calculates the hypersensitivity index and risk grade of the paint based on the skin hypersensitivity index of the base material and the curing agent, and the mixing ratio of the base material and the curing agent, as follows:

Hypersensitivity index of paint = 675*0.8 + 7612.5*0.2 = 2062.5

Risk grade = XI

The controller 20 may determine that the paint is hazardous when the hypersensitivity index of the paint is equal to greater than the reference hypersensitivity index or equal to or greater than the reference risk grade. In this case, the reference hypersensitivity index or the reference risk grade may be set based on animal or alternative animal tests. Hereinafter, the animal or alternative animal test methods for assessing the harmfulness of the paint are described in more detail.

Animal Test

In the present disclosure, the skin irritation and skin corrosiveness of the paint may be verified through the OECD TG404 test. Furthermore, the skin hypersensitivity of the paint may be verified through the OECD TG406 test. The test methods such as OECD TG404 and OECD TG406 are well-known, and a detailed description thereof will be omitted herein.

Table 5 below shows the evaluation of a skin reaction index by assigning scores based on the severity of erythema and edema. Furthermore, the base material or the curing agent may be applied to the animal's skin, and the skin reactions may be observed after 24, 48, and 72 hours. The skin reaction index may be derived at each time point, and the average skin reaction index may be calculated (Equation 2).

[Table 5]

| **Erythema (eschar) / edema** | **Score** |
|---|---|
| No erythema / edema | 0 |
| Very slight erythema / edema | 1 |
| Well-defined erythema / edema | 2 |
| Moderately severe erythema / edema | 3 |

(continued)

| Erythema (eschar) / edema | Score |
|---|---|
| Severe erythema / edema | 5 |

Mean skin response index (mean score) = (skin response index after 24 hours + skin response index after 48 hours + skin response index after 72 hours) / 3 (Equation 2)

Table 6 below presents skin corrosion/irritation risk grades assessment criteria, based on the skin reaction index and the time to onset of skin corrosion or irritation, according to the Globally Harmonized System of Classification and Labeling of Chemicals (GHS, 2019).

[Table 6]

| Category | Assessment criteria |
|---|---|
| 1A | Skin corrosiveness reaction ≤ 3min |
| 1B | Skin corrosiveness reaction ≤ 1hr |
| 1C | Skin corrosiveness reaction ≤ 4hrs |
| 2 | Mean score 2.3 to 4.0 (erythema (eschar) / edema) |
| 3 | Mean score 1.5 to 2.3 (erythema (eschar) / edema) |

The skin corrosiveness of the paint may be assessed by the presence of irreversible damage (skin corrosiveness reaction) to the skin. Typical examples of the skin corrosiveness reactions include skin necrosis, hemorrhagic scabs, etc. As the time the skin corrosiveness reaction is observed on the skin is rapid, it may be assessed that the skin corrosion of the paint is strong.

Preferably, when no skin corrosiveness reaction occurs within 4 hours of the assessment criteria, the paint is considered non-corrosive. When the skin reaction index is less than 1.5, the paint is considered non-irritating.

In the present disclosure, the skin hypersensitivity of the paint may be verified through the OECD TG406 test. To verify the skin hypersensitivity alone, the OECD TG406 test may be conducted at a paint concentration that does not cause the skin corrosiveness/irritation.

Table 7 below shows the Magnusson and Kligman grade for hypersensitivity tests, which is determined based on erythema and edema reactions observed in animals in the OECD TG406 test.

[Table 7]

| Patch test results | Grade |
|---|---|
| No reaction | 0 |
| Scattered erythema observed at application site | 1 |
| Erythema observed over the entire application site | 2 |
| Severe erythema and edema observed over the entire application site | 3 |

Table 8 below shows the skin hypersensitivity grades determined based on the Globally Harmonized System of Classification and Labeling of Chemicals (GHS, 2019).

The Magnuson-Kligman grade of the paint is determined through the OECD TG406 test, and the hypersensitivity rate may be determined by the proportion of test subjects with Magnuson-Kligman grades of 1 to 3 among all test subjects. For example, when 20 animals are tested using the OECD TG406 test and 12 of them have a Magnusson-Kligman grade of 1 to 3, the hypersensitivity rate of the paint is 60%.

In the present disclosure, the skin hypersensitivity may be assessed based on the paint concentration and hypersensitivity rate. Preferably, the paints classified as 1A or 1B in the test results may be assessed to have skin hypersensitivity.

[Table 8]

| Category | Assessment criteria |
|---|---|
| 1A | ≥ 15% hypersensitivity at ≤ 0.2% concentration, or ≥ 60% hypersensitivity at 0.2 to 20% concentration |
| 1B | 15 to 60% hypersensitivity at 0.2 to 20% concentration, or ≥ 15% hypersensitivity at > 20% concentration |

Alternative Animal Test

According to an embodiment of the present disclosure, in the case where animal testing is not feasible due to corporate ethical considerations, a reliable alternative animal test can be conducted to assess the paint's harmfulness.
In an embodiment, the corrosiveness grade of the paint may be checked through the OECD TG 435 test, which replaces the rabbit test. The OECD TG 435 test method is well known, and a detailed description thereof will be omitted herein.
Table 9 below shows criteria for classifying GHS corrosion categories using the OECD TG 435 test, based on the time taken from the moment a test substance is applied to a barrier membrane until the test substance penetrates the barrier membrane.

[Table 9]

| Penetration time | | Category |
|---|---|---|
| Strongly acidic/strongly basic substance | Weakly acidic/weakly basic substance | |
| 0~3min | 0~3min | 1A |
| 3~60min | 3~30min | 1B |
| 60~240min | 3~60min | 1C |
| 240min~ | 60min~ | Non-corrosion |

According to an embodiment of this specification, the skin corrosiveness grade of the paint may be assessed using the OECD TG 435 test. For example, when the paint is non-corrosive, it may be assessed to meet the corrosiveness grade criteria based on an alternative animal test.
When the skin corrosiveness category does not meet the criteria, the paint formulation may be redesigned.
Furthermore, the OECD TG 442B (LLNA) test, which replaces the guinea pig test, may be used to assess the skin hypersensitivity of the paint. The OECD TG 442B (LLNA) test is an experiment in which a test substance is applied to the ears of mice, and the proliferation of lymphocytes in the lymph nodes is measured. The amount of lymphocyte proliferation in the lymph nodes, which is involved in the immune response, is measured using an optical measurement method called the Stimulation Index (SI) value. When the SI value is equal to or greater than a preset SI value (e.g., 1.6), the hypersensitivity may be assessed.
The SI value is a value calculated to assess the potential skin hypersensitivity of the test substance and may be derived using Equation 3 below.

SI value = Test group lymphocyte concentration / Control group lymphocyte concentration (Equation 3)

The irritation/corrosiveness grade and the hypersensitivity grade of the paint may be assessed through the animal or alternative animal test described above, and the reference hypersensitivity index may be set. For example, the reference hypersensitivity index may be set at 1500 points.
Alternatively, for the paint that passed the primary harmfulness criteria by falling below the reference hypersensitivity index, the additional animal or alternative animal test described above may be additionally conducted to assess the harmfulness of secondary paint. When the animal or alternative animal tests indicate that the irritation/corrosiveness grade or hypersensitivity grade of the paint does not meet the criteria, the paint formulation may be redesigned.
In other words, after evaluating the skin harmfulness of the paint based on the skin hypersensitivity index (S250), the animal or alternative animal test may be conducted to select the paint composition (S260). Alternatively, the paint composition may be selected by conducting additional patch test after animal or alternative animal test. Even if the paint is determined to be non-harmful based on the skin hypersensitivity index, when the paint composition is determined to be unsuitable in animal or alternative animal test, or patch test, the paint composition may be reformulated by adjusting the

paint ingredients, amounts, and the mixing ratio of the base material and the curing agent.

In the present disclosure, when the skin irritation/corrosiveness grade of the paint meets the criteria, the additional animal or alternative animal test may be conducted to determine the potential skin hypersensitivity of the paint. While the present specification describes testing to assess the skin corrosion/irritation of the paint as being conducted first, the present disclosure is not limited thereto.

When the hypersensitivity grade of the paint does not meet the criteria based on the assessment criteria, the paint formulation may be redesigned. Furthermore, when the skin irritation/corrosiveness and skin hypersensitivity of the paint meet the criteria, the patch test on workers may be conducted.

Patch Test

When the skin corrosion, irritation, and hypersensitivity of the paint have been verified through the animal or alternative animal test, the patch test or skin diagnostic test may be conducted on workers. The skin diagnostic test is typically a patch test, and skin diagnostic test may be a provocation test to identify allergens in patients with allergic contact dermatitis.

Skin rashes related to irritation or corrosion may be caused either by direct contact with paint (contact irritation) or by a worker's skin hypersensitivity even in the absence of direct contact with the paint (non-contact irritant reaction, allergic reaction). Unlike contact irritation, non-contact irritation is related to the worker's immune system. Therefore, test may help prevent workers at risk of non-contact irritation from working with the paint or strengthen the use of protective equipment during painting.

To identify workers with non-contact irritation, the skin diagnostic test may be conducted in four stages. First, the paint is diluted with a solution such as water, alcohol, acetone, or petroleum jelly to create a sample at an appropriate concentration that will not cause a reaction in normal individuals but only in those with sensitive skin. Preferably, the paint concentration is less than 1%.

Then, the sample is prepared in the form of a patch and applied to a worker, and the worker's skin is observed for 48 hours to determine whether an allergic reaction occurs. The primary assessment is made based on the presence or absence of a reaction. Here, attaching the sample patch to the worker and observing the worker's skin may be performed automatically by a data collection unit, but the present disclosure is not limited thereto.

Since immune responses may occur over time, after the primary assessment, the patch is removed and the area is additionally observed for 48 hours to observe whether a delayed reaction occurs and perform a secondary assessment based thereon.

Finally, the worker's skin is observed for up to 4 days after patch application. When a positive reaction occurs on the worker's skin, it may be assessed as allergic contact dermatitis, and the worker may be assessed to have a non-contact irritation reaction. Table 10 below shows the skin reaction assessment criteria of the International Contact Dermatitis Research Group (ICDRG). When it is 1+ or higher, it may be assessed as a positive reaction.

[Table 10]

| Category | Assessment |
|---|---|
| NT | Not tested |
| ± | Doubtful reaction |
| + | Weak reaction |
| ++ | Strong reaction |
| +++ | Extreme reaction |
| IR | Irritant reaction |

FIG. 7 is a diagram for explaining skin reactions according to the skin reaction assessment criteria of the International Contact Dermatitis Research Group.

Referring to FIG. 7, it may be checked that the skin reactions are clearly displayed in the following order: doubtful reaction, weak reaction, strong reaction, extreme reaction, and irritant reaction.

Through the third skin harmfulness verification step for field workers, the harmfulness of the paint to the skin may be verified thirdly.

In this way, the method for selecting a paint composition according to the present disclosure may evaluate the skin hypersensitivity of the paint by calculating the hypersensitivity index based on the hypersensitivity level of the components in the paint (base material, curing agent) and the amount of components.

According to the method for selecting a paint composition according to the present disclosure, entering the unique numbers and amount of the constituent components of the paint yields the hypersensitivity index and grade, allowing quantitative assessment of the skin hypersensitivity index and grade of the paint.

Therefore, the method solves the problem of paint hypersensitivity not being specifically identified, as it was previously indicated as either hypersensitivity 1 or no hypersensitivity. This method enables accurate assessment of the skin hypersensitivity of the paint during the development and use of the paint.

Furthermore, the method for selecting a paint composition according to the present disclosure may calculate the skin hypersensitivity index and grade of the constituent component of the paint based on the database, and predict the overall skin hypersensitivity of the paint based on the skin hypersensitivity index and the skin hypersensitivity grade.

According to the method for selecting a paint composition according to the present disclosure, the harmfulness of the paint to the skin may be quantitatively and systematically assessed. Based on the evaluation results, the paint may be formulated and used, thereby minimizing the skin problems caused by the paint to the user.

While preferred embodiments of the present disclosure have been described above, the present disclosure is not limited to the above-described embodiments. Those skilled in the art will readily appreciate that modifications made to the embodiments are intended to encompass all modifications deemed equivalent.

Description of Reference Characters

| | | | |
|---|---|---|---|
| 10: | User Input Interface | 20: | Controller |
| 22: | Memory | 30: | User Output Interface |

## Claims

1. A method for selecting a paint composition, comprising: receiving components and amounts of candidate raw materials of the paint composition through a user input interface;

   checking, by a controller, hypersensitivity component information about the candidate raw materials;
   calculating a skin hypersensitivity index of paint based on the components and amounts of the candidate raw materials and the hypersensitivity component information, by considering or not considering a hypersensitivity grade of hypersensitivity components depending on whether the amount of components corresponding to the hypersensitivity components is equal to or greater than a set hypersensitivity amount;
   evaluating, by the controller, harmfulness of the paint to the skin based on the skin hypersensitivity index of the paint; and
   selecting the paint composition based on the evaluated harmfulness of the paint to the skin.

2. The method of claim 1, wherein the paint composition contains a base material and a curing agent.

3. The method of claim 2, further comprising:
   receiving a mixing ratio of the base material and the curing agent.

4. The method of claim 3, wherein the evaluating of the harmfulness of the paint to the skin includes evaluating the harmfulness of the paint to the skin based on the components and amounts of the candidate raw materials, the hypersensitivity component information, and the mixing ratio of the base material and the curing agent.

5. The method of claim 1, further comprising:

   checking corrosiveness component information about the candidate raw materials,
   wherein the calculating of the skin hypersensitivity index of the paint includes:

      when the amount of components corresponding to corrosiveness components based on the corrosiveness component information is equal to or greater than a set corrosive amount, calculating the skin hypersensitivity index of the paint by assigning a weight based on the amount of the corrosiveness component; and
      when the amount of the components corresponding to the corrosiveness components based on the corrosiveness component information is less than a set corrosive amount, calculating the skin hypersensitivity index of the paint without assigning a weight based on the amount of the corrosiveness component.

6. The method of claim 1, wherein the hypersensitivity component information is stored in a database and includes at

least one of information about whether the components correspond to the hypersensitivity components and information about a hypersensitivity grade, and
the hypersensitivity grade includes a high hypersensitivity grade and a low or normal hypersensitivity grade.

**7.** The method of claim 5, wherein the corrosiveness component information is stored in a database and includes at least one of information about whether the components correspond to the corrosiveness components and information about the corrosiveness grade.

**8.** The method of claim 1, wherein the calculating of the skin hypersensitivity index of the paint includes: when the amount of the components corresponding to the hypersensitivity components is less than the set hypersensitivity amount, calculating the skin hypersensitivity index by considering the hypersensitivity grade of the component.

**9.** The method of claim 1, wherein the calculating of the skin hypersensitivity index of the paint includes:

when the amount of the components corresponding to the hypersensitivity component is equal to or greater than the set hypersensitivity amount,
calculating a skin hypersensitivity index by preferentially setting components corresponding to a high hypersensitivity grade in components reaching the set hypersensitivity amount, and considering the hypersensitivity grade of the components reaching the set hypersensitivity amount; and
calculating a skin hypersensitivity index by preferentially setting components corresponding to a low or normal hypersensitivity grade in components exceeding the set hypersensitivity amount without considering the hypersensitivity grade of the components exceeding the set hypersensitivity amount.

**10.** The method of claim 9, wherein the hypersensitivity grade includes a high hypersensitivity grade and a low or normal hypersensitivity grade, and
the skin hypersensitivity index per amount of component corresponding to the high hypersensitivity grade is set to be greater than the skin hypersensitivity index per amount of component corresponding to the low or normal hypersensitivity grade.

**11.** The method of claim 1, wherein the evaluating of the harmfulness of the paint to the skin includes determining the paint as hazardous paint when the skin hypersensitivity index of the paint is equal to or greater than a reference hypersensitivity index, and returning to the receiving of the components and amounts of the candidate raw materials of a new paint composition.

**12.** The method of claim 11, wherein the reference hypersensitivity index is established through animal or an alternative animal test.

**13.** The method of claim 11, wherein the reference hypersensitivity index is set to 1500 points.

**14.** A system for selecting a paint composition, comprising:

a user input interface configured to receive components and amount of candidate raw materials of a paint composition; and
a controller configured to verify hypersensitivity component information of the candidate raw materials, calculate a skin hypersensitivity index of the paint based on the components and amount of the candidate raw materials and the hypersensitivity component information, with or without considering the hypersensitivity grade of the hypersensitivity component, depending on whether the amount of the component corresponding to the hypersensitivity component is equal to or greater than a set hypersensitivity amount, evaluate the harmfulness of the paint to the skin based on the skin hypersensitivity index of the paint, and select a paint composition based on the evaluated harmfulness of the paint to the skin.

**15.** A computer-readable storage medium storing a computer program that causes the method of any one of claims 1 to 13 to be performed on a computer.

**16.** A computer program stored on a computer-readable storage medium, the computer program including executable instructions that, when executed by at least one computer, cause the computer to perform the method of any one of claims 1 to 13.

[Fig. 1]

10
20
30
USER INPUT
INTERFACE
CONTROLLER
MEMORY
USER OUTPUT
INTERFACE
22

[Fig. 2]

START
S110
RECEIVE COMPONENT AND AMOUNT OF CANDIDATE RAW MATERIAL
S120
CHECK HYPERSENSITIVITY COMPONENT INFORMATION OF CANDIDATE RAW MATERIAL
S130
CALCULATE SKIN HYPERSENSITIVITY INDEX
S140
IS HARMFULNESS OF PAINT TO SKIN EVALUATED (IS LESS THAN REFERENCE HYPERSENSITIVITY INDEX?)
NO
YES
S150
SELECT PAINT COMPOSITION
END

[Fig. 3]

START
S200
RECEIVE COMPONENT AND AMOUNT CONSTITUTING BASE MATERIAL AND CURING AGENT OF PAINT AND MIXING RATIO OF BASE MATERIAL AND CURING AGENT
S210
ACQUIRE HYPERSENSITIVITY COMPONENT INFORMATION AND CORROSIVENESS COMPONENT INFORMATION RELATED TO COMPONENT CONSTITUTING BASE MATERIAL AND CURING AGENT
S220
CALCULATE SKIN HYPERSENSITIVITY INDEX OF BASE MATERIAL
S230
CALCULATE SKIN HYPERSENSITIVITY INDEX OF CURING AGENT
S240
CALCULATE SKIN HYPERSENSITIVITY INDEX OF PAINT
S250
EVALUATE HARMFULNESS TO SKIN
S260
SELECT PAINT COMPOSITION
END

[Fig. 4]

S220
CALCULATE SUM OF AMOUNTS OF HYPERSENSITIVE RAW MATERIALS AMONG RAW MATERIALS CONSTITUTING BASE MATERIAL AND SUM OF AMOUNTS OF CORROSIVE RAW MATERIALS
S300
IS SUM OF AMOUNTS OF HYPERSENSITIVE RAW MATERIALS AMONG RAW MATERIALS CONSTITUTING BASE MATERIAL LESS THAN SET HYPERSENSITIVITY AMOUNT?
S310
NO
YES
IS SUM OF AMOUNTS OF CORROSIVE RAW MATERIALS AMONG RAW MATERIALS CONSTITUTING BASE MATERIAL EQUAL TO OR LESS THAN SET CORROSIVE AMOUNT?
S320
NO
IS SUM OF AMOUNTS OF CORROSIVE RAW MATERIALS AMONG RAW MATERIALS CONSTITUTING BASE MATERIAL EQUAL TO OR LESS THAN SET CORROSIVE AMOUNT?
S350
NO
YES
S330
S340
YES
S360
S370
CALCULATE SKIN HYPERSENSITIVITY INDEX OF BASE MATERIAL ACCORDING TO RULE 1
CALCULATE SKIN HYPERSENSITIVITY INDEX OF BASE MATERIAL ACCORDING TO RULE 3
CALCULATE SKIN HYPERSENSITIVITY INDEX OF BASE MATERIAL ACCORDING TO RULE 2
CALCULATE SKIN HYPERSENSITIVITY INDEX OF BASE MATERIAL ACCORDING TO RULE 4
RETURN

[Fig. 5]

S230
CALCULATE SUM OF AMOUNTS OF HYPERSENSITIVE RAW MATERIALS AMONG RAW MATERIALS CONSTITUTING CURING AGENT AND SUM OF AMOUNTS OF CORROSIVE RAW MATERIALS
S400
IS SUM OF AMOUNTS OF HYPERSENSITIVE RAW MATERIALS AMONG RAW MATERIALS CONSTITUTING CURING AGENT LESS THAN SET HYPERSENSITIVITY AMOUNT?
S410
NO
YES
IS SUM OF AMOUNTS OF CORROSIVE RAW MATERIALS AMONG RAW MATERIALS CONSTITUTING CURING AGENT EQUAL TO OR LESS THAN SET CORROSIVE AMOUNT?
S420
NO
YES
IS SUM OF AMOUNTS OF CORROSIVE RAW MATERIALS AMONG RAW MATERIALS CONSTITUTING CURING AGENT EQUAL TO OR LESS THAN SET CORROSIVE AMOUNT?
S450
NO
YES
S430
CALCULATE SKIN HYPERSENSITIVITY INDEX OF CURING AGENT ACCORDING TO RULE 1
S440
CALCULATE SKIN HYPERSENSITIVITY INDEX OF CURING AGENT ACCORDING TO RULE 3
S460
CALCULATE SKIN HYPERSENSITIVITY INDEX OF BASE MATERIAL ACCORDING TO RULE 2
S470
CALCULATE SKIN HYPERSENSITIVITY INDEX OF BASE MATERIAL ACCORDING TO RULE 4
RETURN

[Fig. 6]

PAINT COMPANY
PAINT NAME
CATEGORY
BASE MATERIAL
INPUT ITEM
No.
SUBSTANCE NAME
CAS No.
SUBSTANCE AMOUNT
HYPERSENSITIVITY
CORROSIVENESS
REMARK
WHETHER LIST OF SUBSTANCES IS INCLUDED
SKIN HYPERSENSITIVITY INDEX
0
SKIN HYPERSENSITIVITY GRADE
PAINT COMPANY
PAINT NAME
CATEGORY
BASE MATERIAL
No.
SUBSTANCE NAME
CAS No.
SUBSTANCE AMOUNT
HYPERSENSITIVITY
CORROSIVENESS
REMARK
WHETHER LIST OF SUBSTANCES IS INCLUDED
SKIN HYPERSENSITIVITY INDEX
0.0
SKIN HYPERSENSITIVITY GRADE
BASE MATERIAL
CURING AGENT
MIXED SCORE
MIXED GRADE
Hazard Number
0
0
#DIV/0!
MIXING RATIO
HYPERSENSITIVITY CALCULATION TABLE
Calc
LIST OF SUBSTANCES

[Fig. 7]

| | A | B | C | D | E | F | G | H | I | J |
|---|---|---|---|---|---|---|---|---|---|---|
| 1 | | | | MODIFICATION PROHIBITED | | | | MODIFICATION PROHIBITED | | |
| 2 | | | | 1 | 2 | 3 | 4 | 5 | 6 | |
| 3 | | No. | Substance name | CAS no. | Su | EC no. | Wor | Classifications | Corrosi | group |
| 4 | | 1 | 1,1,1-tris (glycidyloxymethyl) propane | 3454-29-3 | 1,1,1- | 222-384-0 | HS | H360, H314, H317, H411 | 1B | Reactive thinner |
| 5 | | 2 | 1,2,3-propanetriol, polymer with 2- (chloromethyl) oxirane | 25038-04-4 | 1,2,3- | 607-502-4 | HS | H315, H319, H334, H335 | X | Harder, other |
| 6 | | 3 | 1,2-diaminocyclohexane (DCH) | 694-83-7 | 1,2-di | 211-776-7 | HS | H302, H312, H332, H314, H | 1B | Hardeners, cycloaliphatic amines |
| 7 | | 4 | 1,3-bis-aminomethylcyclohexane (1,3-BAC) | 2579-20-6 | 1,3-bi | 219-941-5 | HS | H302, H312, H314, H318, H | 1C | Hardeners, cycloaliphatic amines |
| 8 | | 5 | 1,4-butanediol diglycidyl ether | 2425-79-8 | 1,4-bu | 219-371-7 | HS | H302, H312, H332, H319, H | X | Reactive thinner |
| 9 | | 6 | 1,6-bis (2,3 epoxypropoxy) hexane | 933999-84-9 | 1,6-bi | 618-939-5 | HS | H315, H317, H319, H412 | X | Reactive thinner |
| 10 | | 7 | 1,6-hexanediol diglycidyl ether | 16096-31-4 | 1,6-he | 240-260-4 | HS | H315, H319, H317, H412 | X | Reactive thinner |
| 11 | | 8 | 1- (2-aminoethyl) 2,2-dimethoxy-1-aza-2-silacyclopentane | 618914-51-5 | 1- (2- | 689-749-8 | | H318 | X | Harder, other |
| 12 | | 9 | 1-methoxy-2-propanol | 107-98-2 | 1-met | 203-539-1 | | H226, H336 | X | Solvent |
| 13 | | 10 | 1-phenoxypropan-2-ol | 770-35-4 | 1-phe | 212-222-7 | | H319 | X | Solvent |
| 14 | | 11 | 12-glycidyl oleic acid glycerol ester | 74398-71-3 | 12-gl | 616-085-8 | HS | H315, H317 | X | Fillers, pigments and reinforcing agents |
| 15 | | 12 | 2,2,4-trimethylhexane-1,6-diamine | 25513-64-8 | 2,2,4- | 247-063-2 | HS | H302, H314, H317, H318 | 1B | Hardener, aliphatic amines |
| 16 | | 13 | 2,4,6-tris (dimethylaminomethyl) phenol | 90-72-2 | 2,4,6- | 202-013-9 | GMS | H314, H318 | 1C | Secondary and tertiary amines |
| 17 | | 14 | 2- (2-aminoethoxy) ethanol | 929-06-6 | 2- (2- | 213-195-4 | | H314, H318 | 1C | Solvent |
| 18 | | 15 | 2-ethylhexanoic acid | 149-57-5 | 2-eth | 205-743-6 | | H361d | X | Acids |
| 19 | | 16 | 2-ethylhexyl glycidyl ether | 2461-15-6 | 2-eth | 219-553-6 | HS | H315, H317 | X | Reactive thinner |
| 20 | | 17 | 2-methoxy-1-methylethyl acetate | 108-65-6 | 2-met | 203-603-9 | | H226 | X | Solvent |
| 21 | | 18 | 2-methoxy-1-methylethyl acetate | 108-65-6 | 2-met | 203-603-9 | | H226 | X | Solvent |
| 22 | | 19 | 2-methyl-1,5-pentanediamine (MPMD) | 15520-10-2 | 2-met | 239-556-6 | | H302, H312, H332, H314, H | 1C | Hardener, aliphatic amines |
| 23 | | 20 | 2-methylcyclohexane-1,3-diamine | 13897-56-8 | 2-met | 237-667-4 | | H302, H314, H318 | 1B | Hardeners, cycloaliphatic amines |
| 24 | | 21 | 2-phenoxyethanol | 122-99-6 | 2-phe | 204-589-7 | | H302, H319 | X | Solvent |
| 25 | | 22 | 3,4-epoxycyclohexylmethyl-3,4-epoxycyclohexane carboxylate | 2386-87-0 | 3,4-ep | 219-207-4 | HS | H317 | X | Reactive thinner |
| 26 | | 23 | 3-aminopropyltriethoxysilane | 919-30-2 | 3-ami | 213-048-4 | GMS | H302, H314, H317 | 1B | Harder, other |
| 27 | | 24 | 3-aminopropyltrimethoxysilane | 13822-56-5 | 3-ami | 237-511-5 | | H315, H318 | X | Harder, other |
| 28 | | 25 | 3-cyclohexylaminopropylamine | 3312-60-5 | 3-cycl | 222-001-7 | HS | H302, H314, H318, H317, H | 1B | Hardeners, cycloaliphatic amines |
| 29 | | 26 | 4,4'-diaminocyclohexyl methane | 1761-71-3 | 4,4'-d | 217-168-8 | GMS | H302, H314, H317, H318, H | 1A | Hardeners, cycloaliphatic amines |
| 30 | | 27 | 4,4'-diaminodiphenylmethane | 101-77-9 | 4,4'-d | 202-974-4 | GMS | H373, H317, H341, H350, H | X | Hardener, aromatic amines |
| 31 | | 28 | 4,4'-Isopropylidenediphenol, oligomer with epichlorohydrin and isophoronedia | | 4,4'-Is | 949-140-2 | GMS | H314, H317, H412 | 1B | Hardeners, cycloaliphatic amines |
| 32 | | 29 | 4,4'-Isopropylidenediphenol, oligomeric reaction product with 1 | 113930-69-1 | 4,4'-Is | 500-302-7 | HS | H302, H314, H317, H318, H | 1B | Adduct with polyamines |
| 33 | | 30 | 4,4'-Isopropylidenediphenol, oligomeric reaction products with | 38294-64-3 | 4,4'-Is | 500-101-4 | HS | H314, H317, H318, H412 | 1B | Hardeners, cycloaliphatic amines |
| 34 | | 31 | 4,4'-Isopropylidenediphenol, oligomeric reaction products with | 38294-69-8 | 4,4'-Is | 500-104-0 | HS | H302, H314, H317, H318, H | 1B | Adduct with polyamines |
| 35 | | 32 | 4,4'-methylenebis [N, N-bis (2,3-epoxypropyl) aniline], (TGMDA | 28768-32-3 | 4,4'-m | 249-204-3 | HS | H317, H341, H411 | X | Reactive thinner |

**INTERNATIONAL SEARCH REPORT**

International application No.
**PCT/KR2024/015998**

**A. CLASSIFICATION OF SUBJECT MATTER**

**G16C 60/00**(2019.01)i; **G16H 50/30**(2018.01)i; **G16C 20/30**(2019.01)i; **G16C 20/90**(2019.01)i; **G01N 33/32**(2006.01)i

According to International Patent Classification (IPC) or to both national classification and IPC

**B. FIELDS SEARCHED**

Minimum documentation searched (classification system followed by classification symbols)

G16C 60/00(2019.01); G06Q 10/06(2012.01); G06Q 10/10(2012.01); G06Q 50/26(2012.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 도료(paint), 피부(skin), 과민반응(hypersensitivity), 유해성(harmful), 평가(rating)

**C. DOCUMENTS CONSIDERED TO BE RELEVANT**

| Category* | Citation of document, with indication, where appropriate, of the relevant passages | Relevant to claim No. |
|---|---|---|
| A | RASLAN, R. et al. Safety and health index development for formulated product design: Paint formulation. E3S Web of Conferences. 2019, vol. 90, thesis no. 03002, pp. 1-8. See abstract; and pages 2-6. | 1-16 |
| A | 신용천 등. 조선업의 도장 작업시 취급하는 도료중 유해물질 성분에 관한 연구. 한국산업위생학회지. 1999, vol. 9, no. 1, pp. 156-172 (SHIN, Yong Chul et al. Chemical Composition of Painting materials used in Some Korean Shipyards. Journal of Korean Society of Occupational and Environmental Hygiene.) See pages 158-170. | 1-16 |
| A | KR 10-2021-0119138 A (KOREA UNIVERSITY RESEARCH AND BUSINESS FOUNDATION) 05 October 2021 (2021-10-05) See paragraphs [0029], [0035]-[0037] and [0047]-[0048]. | 1-16 |
| A | 기술지침서. 화학물질의 피부과민성시험 기술지침 (KOSHA GUIDE W-9-2021). 한국산업안전보건공단. 2021, pp. 1-15, non-official translation (Technical Guideline. Technical Guidelines for Skin Sensitization Testing of Chemicals. Korea Occupational Safety and Health Agency.) See pages 1-12. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 January 2025** | **17 January 2025** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/015998**

| **C. DOCUMENTS CONSIDERED TO BE RELEVANT** | | |
|---|---|---|
| Category* | Citation of document, with indication, where appropriate, of the relevant passages | Relevant to claim No. |
| A | KR 10-2022-0086936 A (AMOREPACIFIC CORPORATION et al.) 24 June 2022 (2022-06-24) See paragraphs [0028], [0033], [0074], [0111] and [0159]-[0161]. | 1-16 |

Form PCT/ISA/210 (second sheet) (July 2022)

**INTERNATIONAL SEARCH REPORT**
**Information on patent family members**

International application No.
**PCT/KR2024/015998**

| Patent document cited in search report | Publication date (day/month/year) | Patent family member(s) | Publication date (day/month/year) |
|---|---|---|---|
| KR 10-2021-0119138 A | 05 October 2021 | KR 10-2380047 B1 | 28 March 2022 |
| KR 10-2022-0086936 A | 24 June 2022 | None | |

Form PCT/ISA/210 (patent family annex) (July 2022)